# EUROPEAN PATENT SPECIFICATION

(11) **EP 1 122 250 B1**
(45) Date of publication and mention of the grant of the patent: **26.05.2004**
(21) Application number: 01300950.1
(22) Date of filing: 02.02.2001
(51) Int. Cl.: C07D 311/72, B01D 15/08

(54) **A method of chromotagraphic isolation for vitamin E isomers**
Ein Verfahren zur chromatographischen Isolierung von Isomeren des Vitamins E
Un procédé pour l'isolation des isomères de la vitamin E par chromatographie

(30) Priority: 02.02.2000 MY 0000368
(43) Date of publication of application: 08.08.2001
(73) Proprietor: Malaysian Palm Oil Board, 43000 Kajang, Selangor (MY)
(72) Inventor: May, Choo Yuen, 43000 Kajang, Selangor (MY); Ngan, Ma Ah, 43000 Kajang, Selangor (MY); Basiron, Yusof, 43000 Kajang, Selangor (MY)
(74) Representative: Hayes, Adrian Chetwynd

(56) References cited:
- GB-A- 2 218 989
- US-A- 4 122 094
- US-A- 5 157 132
- T. YARITA ET. AL.: "Supercritical fluid chromatographic determination of tocopherols on an ODS-silica gel column. " JOURNAL OF CHROMATOGRAPHY, A, , vol. 679, 1994, pages 329-34, XP000999044
- G. GALUBA ET. AL.: "Separation of tocopherols and sterols in soybean oil condensate utilising supercritical fluid chromatographs (SFC)" CHEMICAL ANALYSIS (WARSAW), vol. 42, - 1997 pages 245-8, XP000999091
- A. STABY ET. AL. : "Quantitative analysis of marine oils by capillary supercritical fluid chromatography." CHROMATOGRAPHIA, vol. 39, no. 11, December 1994 (1994-12), pages 697-705, XP000999090
- DATABASE WPI Week 9030 Derwent Publications Ltd., London, GB; AN 1990-228702 XP002167717 & JP 02 157274 A (SUMITOMO), 18 June 1990 (1990-06-18)
- E. IBANEZ ET. AL.: "Isolation and separation of tocopherols from olive by-products with supercritical fluids. " JOURNAL OF THE AMERICAN OIL CHEMISTS SOCIETY, vol. 77, no. 2, February 2000 (2000-02), pages 187-90, XP000999629

## Description

### Field of the Invention

The invention relates to a method of chromatographic isolation for Vitamin E isomers, particularly individual Vit E isomers such as α-tocopherol, β-tocopherol, γ-tocopherol δ-tocopherol, α-tocotrienol, β-tocotrienol, γ-tocotrienol, δ-tocotrienol and /or the like from Vit E-comprising compounds such as crude palm oil, palm oil products and/or palm oil by-products, vegetable oils and/or the like.

### Background of the Invention

This invention relates to a process of producing isomeric tocols or Vitamin E isomers ( α-tocopherol, β-tocopherol, γ-tocopherol, δ-tocopherol; and α-tocotrienol, β-tocotrienol, γ-tocotrienol, δ-tocotrienol) from tocols or Vitamin E comprising compounds.

Tocols are Vitamin E and consist of tocopherols (T) and tocotrienols ( (T₃) which are also found in vegetable oils in varying quantity ( see Table A ). The tocols present in most vegetable oils are in the form of tocopherols ( α-, β-, γ- and/or δ-tocopherols ). On the other hand, palm oil is unique as the tocols present are mainly in the form of tocotrienols (α-T₃, γ T₃ and δ- T₃). Both of these tocols have shown interesting physiological properties.

**Table A**

| **Tocopherols determined in vegetable oils (mg/kg)** | | | | | | | |
|---|---|---|---|---|---|---|---|
| Oil | α-T | β-T | γ-T | δ-T | α-T₃ | γ-T3 | δ-T₃ |
| Castor | 28 | 29 | 111 | 310 | - | - | - |
| Cocoa butter | 11 | - | 170 | 17 | 2 | - | - |
| Coconut | - | - | - | 2-4 | 20 | - | - |
| Corn | 134 | 18 | 412 | 39 | - | - | - |
| Cottonseed | 573 | 40 | 317 | 10 | - | - | - |
| Groundnut | 169 | 5 | 144 | 13 | - | - | - |
| Linseed | - | - | - | - | - | - | - |
| Mustard | 75 | - | 494 | 31 | - | - | - |
| Olive | 93 | - | 7 | - | - | - | - |
| Palm from fibres | 1662 | - | - | - | 456 | 485 | 142 |
| Palm | 150 | - | - | - | 1127 | 297 | 80 |
| Palm | 279 | - | 61 | - | 274 | 398 | 69 |
| Rape | 70 | 16 | 178 | 7 | - | - | - |
| Rice bran | 324 | 18 | 53 | - | 236 | 349 | - |
| Safflower | 477 | - | 44 | 10 | - | - | - |
| Sesame | 12 | 6 | 244 | 32 | - | - | - |
| Soyabean | 116 | 34 | 737 | 275 | - | - | - |
| Sunflower | 608 | 17 | 11 | - | - | - | - |
| Wheat germ | 1179 | 398 | 493 | 118 | tr | - | - |

Both the tocopherols and tocotrienols act as powerful nutritional antioxidants and help to reduce cellular damage due to free radicals arising from the body's normal oxidative energy metabolism or from the action of toxic chemicals and pollutants in our environment. Free radicals have been implicated in aging, chronic degenerative diseases and cancer.

Vitamin E is also a natural antioxidants, are present at approximately 600 - 1000 ppm in crude palm oil; the major components being the γ-tocotrienol which has recently been found to have anti-cancer properties besides its known antioxidant activity. Tocotrienols have been found to lower blood cholesterol.

A number of patents have been filed on the recovery of Vitamin E from palm oil and they are:
i) Production of High Concentration of Tocopherols and Tocotrienols from Palm Oil By-Product. US Patent 5,190,618;
ii) Integrated Process for Recovery of Carotenoids and Tocotrienols from oil. US Patent 5,157,132; and
iii) Recovery of Carotenoids, Tocopherols, Tocotrienols,and Sterols from esterified Palm Oil GB 2,218,989.

The first patent discloses the extraction of Vitamin E from palm fatty acid distillates (PFAD) which involves conversion of a PFAD into methyl esters, distillation of methyl esters followed by ion-exchange chromatography and molecular distillation to get Vitamin E concentrate of more than 90%.
The second patent discloses extraction of Vitamin E from crude palm oil (CPO) by converting CPO to methyl esters. Thereafter remove the esters by molecular distillation to yield carotene-rich and vitamin E-rich fractions.

The third patent discloses conversion of crude palm oil into methyl esters followed by subjecting the methyl esters in the presence of solvents into chromatographic separation with an adsorbent to yield carotene-rich, vitamin E-rich and sterol-rich fractions.

The above three inventions only describe the isolation of vitamin E from palm oil and do not include any work on the isolation of individual vitamin E isomers, which have exhibit important physiological properties.

Journal of Chromatography, A. Vol. 679, p 329-34 (1994) and Chemical Analysis (Wersaw) vol. 42, p 245-8 (1997) describe processes for separating tocopherols using supercritical fluid chromatography (SFC). Chromatographic Vol. 39, No. 11/12, December 1994 describes a process for the quantitative analysis of marine oils. GB 2,218,989 describes a method of recovery of tocopherols and tocotrienols from esterified palm oil. The latter document, however, is silent on the use of SFC.

Hence, there is a need to provide a separation, particularly an isolation method for Vitamin E isomers which avoids or eliminates the sole use of solvents and consequently, rendering the process "non-hazardous".

### Summary of the Invention

In accordance with the present invention, there is provided a method of chromatographic isolation for Vitamin E (Vit E) isomers as defined in claim 1.

In particular, the present invention stipulates the use of supercritical fluid ( such as SC-CO₂) in combination with adsorbents ( such as silica gel or C18 reverse phase silica gel) to effect adsorption and/or desorption of Vit E isomers from Vit-E comprising compounds.

Most preferably, the present invention is subsequently applied for isolating Vit E isomers from;
a) crude vegetable oil esters ( via catalytic alcoholic esterification/trans-esterification of vegetable oils ) alone or by distilling the esters or by distilling the esters followed by saponification to yield vitamin E concentrate,
   OR
b) unsaponifiable matters of vegetable oils and fats ( through saponification process)
   OR
c) Vitamin E concentrate obtained through any/various processes including chromatography ( solvent and supercritical ) as well as supercritical fluid extraction.

A main advantage of the invention lies in the use of liquefied gas at supercritical conditions, hence avoiding or reducing the prior requirements for hazardous solvents.

### Detailed description of the Invention

The method of chromatographic isolation of Vitamin E isomers from e.g. crude palm oil (CPO), palm oil products and/or palm oil by-products, vegetable oils and/or Vitamin E-comprising compounds, inclues :
(I) Introducing the Vitamin E comprising-compounds onto selected adsorbents to effect adsorption of Vitamin E isomers; and
(II) subsequently desorbing the adsorbed Vitamin E isomers
wherein,
the adsorption/desorption of the vitamin E isomers are carried out under a supercritical fluid environment.

Vit E isomers may be isolated from Vit E concentrate as derived through,
(i) Esterifying the free fatty acid component of the Vit. E isomers-comprising oils with one or more monohydric alcohols into esters. The esterification may be carried out via employing (a) a solid alkali metal bisulfate, a sulfate acid and/or a strongly acidic-ion exchange resin or any acid as a catalyst;
(ii) Trans-esterifying the glyceride components of Vit. E isomers-comprising oils with one or more monohydric alcohols ( C₁ to C₈ into monoesters.
   The trans-esterification may be carried out via employing a basic catalyst. [Both steps i and ii may be carried out via employing an enzyme e.g. candida rugosa and/or the like effective catalyst];
(iii) Removing the formed esters in step (i-ii) by vacuum distillation or molecular distillation thereby yielding vitamin E concentrate. The esters may be removed via vacuum distillation or molecular distillation.
OR
Supercritical extraction and/or any chromatographic methods which are effective in extracting Vitamin E concentrate from vegetable oils.

Alternatively, the present invention may be applied to unsaponifiable materials as obtained through saponification of vegetable oils.

**Adsorbents**: Selective adsorbent for the adsorption of the Vitamin E isomers comprise silica gel, C18 reverse phase silica gel and/or the like which are equivalently effective in adsorbing Vit E isomers from any Vit E comprising-compounds.

Solvents: The solvents employed for the present method comprises;
i) supercritical fluid, e.g. SC-CO₂ ( 100% ). Other applicable solvents include propane, propene, ethylene and/or the like;
   OR
ii) supercritical fluid ( e.g. SC-CO₂) in combination with an entrainer (solvent such as alcohol and/or the like organic solvent)

**Operating conditions** Individual Vitamin E isomers may be isolated with substantially higher purity (e.g. > 50%) in a single run by varying pressures from 50 kg/cm² to 600 kg/cm² and temperatures 30°C -100°C.

It shall therefore be illustrated, by means of the following examples, that supercritical fluid applied to solid adsorbents (silica gel and/or C18 reverse phase silica gel), provides a very satisfactory means for isolating Vitamin E isomers ( α-T, β-T, γ-T, δ-T and α-T₃, β-T₃, γ-T₃ and δ-T₃) from CPO (crude palm oil), palm oil products, vegetable oil-and/or the like Vitamin E comprising compounds.

The Vitamin E isomers recovered are useful in pharmaceutical compositions, food and health food formulations, dietary supplements and cosmetics formulations. They can also be used as fine chemicals and standard reference materials.

### Example 1: Isolation of Palm Vitamin E isomers

Crude palm oil (containing 600- 1000 ppm of vitamin E) was trans-esterified into methyl esters. The methyl esters were removed through molecular distillation at a temperature of 90-180 degree C followed by saponification to yield a vitamin E concentrate ( > 50% ).
Vit. E concentrate (0.01g; concentration: ~50%) was dissolved in organic solvent, such as dichloromethane or ethanol and loaded onto a column (silica gel, with internal diameter 20mm and length 250mm). The Vitamin E concentrate mixture was eluted using a mixture of supercritical carbon dioxide (5ml, 95.2%) and an entrainer, ethanol ( 0.25ml, 4.8%) with a flowrate of 5.25ml/min. The pressure of the supercritical fluid chromatography system was kept constant right through the run at P=180kg/cm², keeping temperature constant at 70°C. The four vitamin E isomers were collected at different time intervals and are shown in Table 1. The presence and concentration of Vitamin E isomer were confirmed by HPLC.

### Example 2

The procedure of example 1 was repeated except vitamin E concentrate obtained from palm fatty acid distillate was used. The results are shown in Table 2.

### Example 3

The procedure of example 1 was repeated except palm pressed fiber oil vitamin E concentrate was used. The results are shown in Table 3.

### Example 4

The procedure of example 1 was repeated except soybean oil vitamin E concentrate was used. The results are shown in Table 4.

### Example 5

The procedure of example 1 was repeated except corn oil vitamin E concentrate was used. The results are shown in Table 5.

### Example 6

The procedure of example 1 was repeated except vitamin E from Canola (rapeseed oil) oil was used. The results are shown in Table 6.

### Example 7

The procedure of example 1 was repeated except vitamin E from olive oil was used. The results are shown in Table 7.

### Example 8

The procedure of example 1 was repeated except vitamin E from cottonseed oil was used. The results are shown in Table 8.

### Example 9

The procedure of example 1 was repeated except vitamin E from groundnut oil was used. The results are shown in Table 9.

### Example 10

The procedure of example 1 was repeated except vitamin E from ricebran oil was used. The results are shown in Table 10.

### Example 11

The procedure of example 1 was repeated except vitamin E from sunflower seed oil was used. The results are shown in Table 11.

### Example 12

The procedure of example 1 was repeated except C18 reverse phase silica gel was used as adsorbent instead of silica gel. Other conditions used are : Pressure: 180 kg/cm2, CO2: ethanol ratio 93.75% : 6.25%. The four vitamin E isomers ( alpha-tocopherol, alpha-tocotrienol, gamma-tocotrienol and delta-tocotrienol) were eluted in the same elution order as in example 1 and collected in high purity as in example 1. The same procedures using C18 reverse phase silica gel as adsorbents were repeated for other palm oil products, palm oil by-products and other vegetable oils as mentioned in Examples 2-11. Similar elution order of vitamin E isomers was observed for both types of adsorbents used.

**Table 1:**

| Isolation of Palm Vit.E isomers (α-T, α-T₃, γ-T₃ and δ-T₃) | | | | | |
|---|---|---|---|---|---|
| Fractions | Collection Time (min) | Vit.E α-T | Vit.E α-T₃ | Vit.E γ-T₃ | Vit.E δ-T₃ |
| S/M | - | 12.7 | 11.4 | 19 | 5.8 |
| 1 | - | - | - | - | - |
| 2 | 41-47 | 100 | - | - | - |
| 3 | 47-54 | - | 100 | - | - |
| 4 | 54-61 | - | - | 100 | - |
| 5 | 61-77 | - | - | 99 | 1 |
| 6 | 77-145 | - | - | 3 | 97 |
| 7 | 145- | - | - | - | - |
| a. S/M : Starting Material (vit. E concentrate ~50%) | | | | | |
| b. T: tocopherol | | | | | |
| c. T₃: tocotrienol | | | | | |
| d. Conditions: P=180kg/cm², T= 70°C | | | | | |
| e. SC-CO₂: ethanol = 5ml:0.25ml (95.2% : 4.8%) | | | | | |

**Table 2:**

| Isolation of Palm Vit.E isomers (α-T, α-T₃, γ-T₃ and δ-T₃) from Palm Fatty Acid Distillate | | | | | |
|---|---|---|---|---|---|
| Fractions | Collection Time (min) | Vit.E α-T | Vit.E α-T₃ | Vit.E γ-T₃ | Vit.E δ-T₃ |
| S/M | - | 22.7 | 21.4 | 39 | 9.8 |
| 1 | | | | | |
| 2 | 41-47 | 100 | | | |
| 3 | 47-54 | | 100 | | |
| 4 | 54-61 | | | 100 | |
| 5 | 61-77 | | | 98 | 2 |
| 6 | 77-145 | | | 2 | 98 |
| 7 | 145- | | | | |
| a. S/M : Starting Material (vit. E concentrate ~90%) | | | | | |
| b. T: tocopherol | | | | | |
| c. T₃: tocotrienol | | | | | |
| d. Conditions: P=180kg/cm², T= 70°C | | | | | |
| e. SC-CO₂: ethanol = 5ml:0.25ml (95.2% : 4.8%) | | | | | |

**Table 3:**

| Isolation of Palm Vit.E isomers (α-T, α-T₃, γ-T₃ and δ-T₃) from Palm Pressed Fibre Oil | | | | | |
|---|---|---|---|---|---|
| Fractions | Collection Time (min) | Vit.E α-T | Vit.E α-T₃ | Vit.E γ-T₃ | Vit.E δ-T₃ |
| S/M | - | 42.7 | 11.4 | 29 | 10.8 |
| 1 | | | | | |
| 2 | 41-47 | 100 | | | |
| 3 | 47-54 | | 100 | | |
| 4 | 54-61 | | | 100 | |
| 5 | 61-77 | | | 99 | 1 |
| 6 | 77-145 | | | 2.2 | 97.8 |
| 7 | 145- | | | | |
| a. S/M: Starting Material (vit. E concentrate ~90%) | | | | | |
| b. T: tocopherol | | | | | |
| c. T₃: tocotrienol | | | | | |
| d. Conditions: P=180kg/cm², T= 70° | | | | | |
| e. SC-CO₂: ethanol = 5ml:0.25ml (95.2%: 4.8%) | | | | | |

**Table 4:**

| Isolation of P Vit.E isomers (α-T, β-T, γ-T and δ-T) from Sovabean Oil | | | | | |
|---|---|---|---|---|---|
| Fractions | Collection Time (min) | Vit.E α-T | Vit.E β-T | Vit.E γ-T | Vit.E δ-T |
| S/M | - | 11.6 | 3.4 | 53.7 | 17.5 |
| 1 | | | | | |
| 2 | 35-41 | 100 | | | |
| 3 | 42-47 | | 99 | | |
| 4 | 48-55 | | | 100 | |
| 5 | 58-65 | | | | 100 |
| a. S/M : Starting Material (vit. E concentrate ~80%) | | | | | |
| b. T: tocopherol | | | | | |
| c. Conditions: P=180kg/cm², T= 70°C | | | | | |
| d. SC-CO₂ : ethanol = 5ml:0.25ml (95.2%: 4.8%) | | | | | |

**Table 5:**

| Isolation of Vit.E isomers (α-T, β-T, γ-T and δ-T) from Corn Oil | | | | | |
|---|---|---|---|---|---|
| Fractions | Collection Time (min) | Vit.E α-T | Vit.E β-T | Vit.E γ-T | Vit.E δ-T |
| S/M | - | 23.4 | 1.8 | 51.2 | 3.9 |
| 1 | | | | | |
| 2 | 35-41 | 100 | | | |
| 3 | 42-47 | | 92 | | |
| 4 | 48-55 | | | 100 | |
| 5 | 58-65 | | | | 92 |
| a. S/M: Starting Material (vit. E concentrate ~80%) | | | | | |
| b. T: tocopherol | | | | | |
| c. Conditions: P=180kg/cm², T= 70°C | | | | | |
| d. SC-CO₂: ethanol = 5ml:0.25ml (95.2%: 4.8%) | | | | | |

**Table 6:**

| Isolation of Vit.E isomers (α-T, β-T, γ-T and δ-T) from Canola Oil | | | | | |
|---|---|---|---|---|---|
| Fractions | Collection Time (min) | Vit.E α-T | Vit.E β-T | Vit.E γ-T | Vit.E δ-T |
| S/M | | 20 | 8 | 58 | 2 |
| 1 | | | | | |
| 2 | 35-41 | 100 | | | |
| 3 | 42-47 | | 85 | | |
| 4 | 48-55 | | | 100 | |
| 5 | 58-65 | | | | 80 |
| a. S/M : Starting Material (vit. E concentrate ~90%) | | | | | |
| b. T: tocopherol | | | | | |
| c. Conditions: P=180kg/cm², T= 70°C | | | | | |
| d. SC-CO₂ : ethanol=5ml:0.25ml (95.2%: 4.8%) | | | | | |

**Table 7:**

| Isolation of Vit.E isomers (α-T, β-T, γ-T and δ-T) from Olive Oil | | | | | |
|---|---|---|---|---|---|
| Fractions | Collection Time (min) | Vit.E α-T | Vit.E β-T | Vit.E γ-T | Vit.E δ-T |
| S/M | - | 83 | 7 | | |
| 1 | | | | | |
| 2 | 35-41 | 100 | | | |
| 3 | 42-47 | | 80 | | |
| 4 | 48-55 | | | | |
| 5 | 58-65 | | | | |
| a. S/M: Starting Material (vit. E concentrate ~90%) | | | | | |
| b. T: tocopherol | | | | | |
| c. Conditions: P=180kg/cm², T= 70°C | | | | | |
| d. SC-CO₂: ethanol = 5ml:0.25ml (95.2% : 4.8%) | | | | | |

**Table 8:**

| Isolation of Vit.E isomers (α-T, β-T, γ-T and δ-T) from Cottonseed Oil | | | | | |
|---|---|---|---|---|---|
| Fractions | Collection Time (min) | Vit.E α-T | Vit.E β-T | Vit.E γ-T | Vit.E δ-T |
| S/M | - | 57.3 | 4 | 31.7 | 1 |
| 1 | | | | | |
| 2 | 35-41 | 100 | | | |
| 3 | 42-47 | | 92 | | |
| 4 | 48-55 | | | 100 | |
| 5 | 58-65 | | | | 85 |
| a. S/M : Starting Material (vit. E concentrate ~90%) | | | | | |
| b. T: tocopherol | | | | | |
| c. Conditions: P=180kg/cm², T= 70°C | | | | | |
| d. SC-CO₂: ethanol = 5ml:0.25ml (95.2% 4.8%) | | | | | |

**Table 9:**

| Isolation of Vit.E isomers (α-T, β-T, γ-T and δ-T) from Groundnut oil | | | | | |
|---|---|---|---|---|---|
| Fractions | Collection Time (min) | Vit.E α-T | Vit.E β-T | Vit.E γ-T | Vit.E δ-T |
| S/M | - | 42.9 | 1.5 | 34.4 | 3 |
| 1 | | | | | |
| 2 | 35-41 | 100 | | | |
| 3 | 42-47 | | 80 | | |
| 4 | 48-55 | | | 100 | |
| 5 | 58-65 | | | | 85 |
| a. S/M : Starting Material (vit. E concentrate ~80%) | | | | | |
| b. T: tocopherol | | | | | |
| c. Conditions: P=180kg/cm², T= 70°C | | | | | |
| d. SC-CO₂: ethanol = 5ml:0.25ml (95.2% : 4.8%) | | | | | |

**Table 10:**

| Isolation of Vit.E isomers (α-T, γ-T, α-T3 and γ-T3) from Ricebran Oil | | | | | |
|---|---|---|---|---|---|
| Fractions | Collection Time (min) | Vit.E α-T | Vit.E γ-T | Vit.E α-T3 | Vit.E γ-T3 |
| S/M | - | 28.2 | 5 | 20.6 | 38.5 |
| 1 | | | | | |
| 2 | 35-41 | 100 | | | |
| 3 | 42-47 | | 85 | | |
| 4 | 48-55 | | | 100 | |
| 5 | 58-65 | | | | 100 |
| a. S/M : Starting Material (vit. E concentrate ~90%) | | | | | |
| b. T: tocopherol | | | | | |
| c. T3: tocotrienol | | | | | |
| d. Conditions: P=180kg/cm², T= 70°C | | | | | |
| e. SC-CO₂: ethanol = 5ml:0.25ml (95.2% : 4.8%) | | | | | |

**Table 11:**

| Isolation of Vit.E isomers (α-T, β-T, γ-T and δ-T) from Sunflowerseed Oil | | | | | |
|---|---|---|---|---|---|
| Fractions | Collection Time (min) | Vit.E α-T | Vit.E β-T | Vit.E γ-T | Vit.E δ-T |
| S/M | - | 80.8 | 3.3 | 1.1 | |
| 1 | | | | | |
| 2 | 35-41 | 100 | | | |
| 3 | 42-47 | | 85 | | |
| 4 | 48-55 | | | 85 | |
| 5 | 58-65 | | | | |
| a. S/M : Starting Material (vit. E concentrate ~80%) | | | | | |
| b. T: tocopherol | | | | | |
| c. Conditions: P=180kg/cm², T= 70°C | | | | | |
| d. SC-CO₂: ethanol = 5ml:0.25ml (95.2% : 4.8%) | | | | | |

## Claims (Claims for the following Contracting State(s): FR)

1. A method of chromatographic isolation of Vitamin E isomers from compounds comprising Vitamin E isomers, said method comprising the steps of:
(i) esterifying free fatty acid components of said compounds into esters with one or more monohydric alcohols;
(ii) transesterifying glyceride components of said compounds into esters with one or more monohydric alcohols;
(iii) introducing the product mixture from steps (i) and (ii) onto selective adsorbents to effect adsorption of Vitamin E isomers; and subsequently
(iv) effecting an individual desorption of the adsorbed Vitamin E isomers, wherein the adsorption and/or desorption of the Vitamin E isomers are carried out under a supercritical fluid environment.

2. A method of chromatographic isolation of claim 1, wherein the selective adsorbents include silica gel and/or reverse phase C18 silica gel.

3. A method of chromatographic isolation of claim 1, wherein the adsorption/desorption of the Vit E isomers are carried out under a supercritical fluid environment comprising an effective amount of CO₂ as a solvent.

4. A method of chromatographic isolation of claim 1, wherein the adsorption/desorption of the Vit E isomers are carried out under a supercritical fluid environment comprising an effective amount of a combination of CO₂ and an entrainer.

5. A method of chromatographic isolation of claim 4, wherein the entrainer comprises an alcohol.

6. A method of chromatographic isolation of any one of claims 3-5 wherein the supercritical fluid conditions comprise operating temperatures of T=30-100°C and pressures of P=50-600 kg/cm².

7. A method of chromatographic isolation of Vitamin E isomers according to claim 1, wherein the esters formed in steps (i) and (ii) are removed to form a Vitamin E concentrate,
wherein the substances which form the source of the Vitamin E isomers introduced into the selective adsorbents comprise said Vitamin E concentrate.

8. A method of chromatographic isolation of claim 1, wherein:
step (i) is carried out via employing a solid alkali metal bisulfate, a sulfate acid and/or a strongly acidic-ion exchange resin as a catalyst;
step (ii) is carried out via employing a basic catalyst;
or wherein
the esterification and/or trans-esterification are carried out via employing an enzyme.

9. A method of chromatographic isolation of claim 1, wherein the esterification and/or trans-esterification comprises the use of one or more C₁-C₈ alcohols.

10. A method of chromatographic isolation of claim 9 wherein the esterification and/or trans-esterification comprises the use of methanol.

11. A method of chromatographic isolation of claim 5 or 6 wherein the entrainer comprises an alkyl alcohol.

12. A method of chromatographic isolation of Vitamin E isomers according to claim 1 wherein the compounds comprising Vitamin E include crude vegetable oil, vegetable oil products and/or vegetable oil by-products.

13. A method of chromatographic isolation of Vitamin E isomers according to claim 1, said method incorporating additional steps prior to adsorption and desorption stages under the supercritical fluid environment, said additional steps comprising the steps of:
(a) removing the esters from the product mixture of steps (i) and (ii) ,
(b) saponifying the fraction of the product mixture of steps (i) and (ii), which fraction contains the non-esterified components;
(c) removing the saponified products, and thereby yielding a fraction containing non-saponifiable products;
wherein the substances, which form the source of Vitamin E isomers, introduced onto the selective adsorbents, comprise the fraction containing the non-saponifiable products from step (c) above.

14. A method of chromatographic isolation of Vitamin E isomers according to claim 1, said method incorporating the additional step(s) of concentrating the compounds comprising Vitamin E through any or various processes including chromatography involving the use of the supercritical fluid environment.

15. A method of chromatographic isolation of Vitamin E isomers according to claim 1, said method incorporating the additional step(s) of extracting a Vitamin E concentrate from the compounds comprising Vitamin E through any or various processes including chromatographic isolation involving the use of supercritical fluid environment.

16. A method of chromatographic isolation of Vitamin E isomers according to claim 1, which is employed to separate Vitamin E isomers from soya bean oil, corn oil, canola oil, olive oil, cottonseed oil and/or sunflowerseed oil.

17. A method of chromatographic isolation of Vitamin E isomers according to claim 16, wherein said Vitamin E isomers are separated into α-T, β-T, γ-T and δ-T isomers.

18. A method of chromatographic isolation of Vitamin E isomers according to claim 1, which is employed to separate Vitamin E isomers from groundnut oil.

19. A method of chromatographic isolation of Vitamin E isomers according to claim 18, wherein the Vitamin E isomers are separated into α-T, β-T, γ-T and δ-T isomers.

20. A method of chromatographic isolation of Vitamin E isomers according to claim 1, which is employed to separate Vitamin E isomers from ricebran oil.

21. A method of chromatographic isolation of Vitamin E isomers according to claim 20, wherein the Vitamin E isomers are separated into α-T, γ-T, α-T₃ and γ-T₃ isomers.

22. A method of chromatographic isolation of Vitamin E isomers according to claim 1, which is employed to separate Vitamin E isomers from crude palm oil, palm fatty acid distillate, and/or palm pressed fibre oil.

23. A method of chromatographic isolation of Vitamin E isomers according to claim 22, wherein the Vitamin E isomers are separated into α-T, γ-T₃, δ-T₃ and α-T₃ isomers.

24. A method of chromatographic isolation of Vitamin E isomers according to claim 5, wherein the alcohol is ethanol.

25. A method of chromatographic isolation of Vitamin E isomers according to claim 1 wherein the supercritical solvent used is propane, propene or ethylene.

26. A method of chromatographic isolation as claimed in claim 1, wherein the esterified and transesterified products of steps (i) and (ii) are removed by vacuum distillation, prior to steps (iii) and (iv).

## Claims (Claims for the following Contracting State(s): DE, CH, GB)

1. A method of chromatographic isolation of Vitamin E isomers from compounds comprising Vitamin E isomers, said method comprising the steps of:
(i) esterifying free fatty acid components of said compounds into esters with one or more monohydric alcohols;
(ii) transesterifying glyceride components of said compounds into esters with one or more monohydric alcohols;
(iii) introducing the product mixture from steps (i) and (ii) onto selective adsorbents to effect adsorption of Vitamin E isomers; and subsequently
(iv) effecting an individual desorption of the adsorbed Vitamin E isomers, wherein the adsorption and/or desorption of the Vitamin E isomers are carried out under a supercritical fluid environment,
wherein compounds comprising Vitamin E isomers are not crude palm oils and/or palm oil products.

2. A method of chromatographic isolation of claim 1, wherein the selective adsorbents include silica gel and/or reverse phase C18 silica gel.

3. A method of chromatographic isolation of claim 1, wherein the adsorption/desorption of the Vit E isomers are carried out under a supercritical fluid environment comprising an effective amount of CO₂ as a solvent.

4. A method of chromatographic isolation of claim 1, wherein the adsorption/desorption of the Vit E isomers are carried out under a supercritical fluid environment comprising an effective amount of a combination of CO₂ and an entrainer.

5. A method of chromatographic isolation of claim 4, wherein the entrainer comprises an alcohol.

6. A method of chromatographic isolation of any one of claims 3-5 wherein the supercritical fluid conditions comprise operating temperatures of T=30-100°C and pressures of P=50-600 kg/cm².

7. A method of chromatographic isolation of Vitamin E isomers according to claim 1, wherein the esters formed in steps (i) and (ii) are removed to form a Vitamin E concentrate,
wherein the substances which form the source of the Vitamin E isomers introduced into the selective adsorbents comprise said Vitamin E concentrate.

8. A method of chromatographic isolation of claim 1, wherein:
step (i) is carried out via employing a solid alkali metal bisulfate, a sulfate acid and/or a strongly acidic-ion exchange resin as a catalyst;
step (ii) is carried out via employing a basic catalyst;
or wherein
the esterification and/or trans-esterification are carried out via employing an enzyme.

9. A method of chromatographic isolation of claim 1, wherein the esterification and/or trans-esterification comprises the use of one or more C₁-C₈ alcohols.

10. A method of chromatographic isolation of claim 9 wherein the esterification and/or trans-esterification comprises the use of methanol.

11. A method of chromatographic isolation of claim 5 or 6 wherein the entrainer comprises an alkyl alcohol.

12. A method of chromatographic isolation of Vitamin E isomers according to claim 1 wherein the compounds comprising Vitamin E include crude vegetable oil, vegetable oil products and/or vegetable oil by-products.

13. A method of chromatographic isolation of Vitamin E isomers according to claim 1, said method incorporating additional steps prior to adsorption and desorption stages under the supercritical fluid environment, said additional steps comprising the steps of:
(a) removing the esters from the product mixture of steps (i) and (ii),
(b) saponifying the fraction of the product mixture of steps (i) and (ii), which fraction contains the non-esterified components;
(c) removing the saponified products, and thereby yielding a fraction containing nonsaponifiable products;
wherein the substances, which form the source of Vitamin E isomers, introduced onto the selective adsorbents, comprise the fraction containing the non-saponifiable products from step (c) above.

14. A method of chromatographic isolation of Vitamin E isomers according to claim 1, said method incorporating the additional step(s) of concentrating the compounds comprising Vitamin E through any or various processes including chromatography involving the use of the supercritical fluid environment.

15. A method of chromatographic isolation of Vitamin E isomers according to claim 1, said method incorporating the additional step(s) of extracting a Vitamin E concentrate from the compounds comprising Vitamin E through any or various processes including chromatographic isolation involving the use of supercritical fluid environment.

16. A method of chromatographic isolation of Vitamin E isomers according to claim 1, which is employed to separate Vitamin E isomers from soya bean oil, corn oil, canola oil, olive oil, cottonseed oil and/or sunflowerseed oil.

17. A method of chromatographic isolation of Vitamin E isomers according to claim 16, wherein said Vitamin E isomers are separated into α-T, β-T, γ-T and δ-T isomers.

18. A method of chromatographic isolation of Vitamin E isomers according to claim 1, which is employed to separate Vitamin E isomers from groundnut oil.

19. A method of chromatographic isolation of Vitamin E isomers according to claim 18, wherein the Vitamin E isomers are separated into α-T, β-T, γ-T and δ-T isomers.

20. A method of chromatographic isolation of Vitamin E isomers according to claim 1, which is employed to separate Vitamin E isomers from ricebran oil.

21. A method of chromatographic isolation of Vitamin E isomers according to claim 20, wherein the Vitamin E isomers are separated into γ-T, α-T, α-T₃ and γ-T₃ isomers.

22. A method of chromatographic isolation of Vitamin E isomers according to claim 1, wherein the supercritical solvent used in propane, propene or ethylene.

23. A method of chromatographic isolation of Vitamin E isomers according to claim 5, wherein the alcohol is ethanol.

24. A method of chromatographic isolation as claimed in claim 1, wherein the esterified and transesterified products of steps (i) and (ii) are removed by vacuum distillation, prior to steps (iii) and (iv).

## Patentansprüche (Patentansprüche für folgende(n) Vertragsstaat(en): FR)

1. Verfahren zur chromatographischen Isolierung von Vitamin E-Isomeren aus Verbindungen, welche Vitamin E-Isomere umfassen, wobei das Verfahren die Schritte umfasst:
(i) Verestern von freien Fettsäurebestandteilen der Verbindungen mit einem oder mehreren einwertigen Alkoholen zu Estern;
(ii) Umestern von Glyceridbestandteilen der Verbindungen mit einem oder mehreren einwertigen Alkoholen zu Estern;
(iii) Einbringen des Produktgemischs aus den Schritten (i) und (ii) auf selektive Adsorbtionsmittel, um die Adsorption der Vitamin E-Isomere zu bewirken; und anschließend
(iv) Ausführen einer individuellen Desorption der adsorbierten Vitamin E-Isomere, worin die Adsorption und/oder Desorption der Vitamin E-Isomere im Milieu eines überkritischen Fluids durchgeführt werden.

2. Verfahren zur chromatographischen Isolierung nach Anspruch 1, worin die selektiven Adsorptionsmittel Silicagel und/oder Umkehrphasen-C18-Silicagel umfassen.

3. Verfahren zur chromatographischen Isolierung nach Anspruch 1, worin die Adsorption/Desorption der Vitamin E-Isomere im Milieu eines überkritischen Fluids durchgeführt werden, welches eine wirksame Menge CO₂ als ein Lösungsmittel umfasst.

4. Verfahren zur chromatographischen Isolierung nach Anspruch 1, worin die Adsorption/Desorption der Vitamin E-Isomere im Milieu eines überkritischen Fluids durchgeführt werden, welches eine wirksame Menge einer Kombination von CO₂ und einem Schleppmittel umfasst.

5. Verfahren zur chromatographischen Isolierung nach Anspruch 4, worin das Schleppmittel einen Alkohol umfasst.

6. Verfahren zur chromatographischen Isolierung nach einem der Ansprüche 3-5, worin die Bedingungen des überkritischen Fluids Betriebstemperaturen von T=30-100°C und Drücke von P=50-600 kg/cm² umfassen.

7. Verfahren zur chromatographischen Isolierung von Vitamin E-Isomeren nach Anspruch 1, worin die in den Schritten (i) und (ii) gebildeten Ester entfernt werden, um ein Vitamin E-Konzentrat zu bilden,
worin die Substanzen, welche die Quelle der Vitamin E-Isomere bilden, welche in die selektiven Adsorptionsmittel eingebracht werden, das Vitamin E-Konzentrat umfassen.

8. Verfahren zur chromatographischen Isolierung nach Anspruch 1, worin:
Schritt (i) mittels Verwendung eines festen Alkalimetallbisulfats, einer Sulfatsäure und/oder eines stark sauren Ionenaustauschharzes als ein Katalysator durchgeführt wird;
Schritt (ii) mittels Verwendung eines basischen Katalysators durchgeführt wird;
oder worin die Veresterung und/oder Umesterung mittels Verwendung eines Enzyms durchgeführt werden.

9. Verfahren zur chromatographischen Isolierung nach Anspruch 1, worin die Veresterung und/oder Umesterung die Verwendung von einem oder mehreren C₁-C₈-Alkoholen umfasst.

10. Verfahren zur chromatographischen Isolierung nach Anspruch 9, worin die Veresterung und/oder Umesterung die Verwendung von Methanol umfasst.

11. Verfahren zur chromatographischen Isolierung nach Anspruch 5 oder 6, worin das Schleppmittel einen Alkylalkohol umfasst.

12. Verfahren zur chromatographischen Isolierung von Vitamin E-Isomeren nach Anspruch 1, worin die Verbindungen, welche Vitamin E umfassen, rohes Pflanzenöl, Pflanzenölprodukte und/oder Pflanzenölnebenprodukte einschließen.

13. Verfahren zur chromatographischen Isolierung von Vitamin E-Isomeren nach Anspruch 1, wobei das Verfahren vor den Adsorptions- und Desorptionsschritten im Milieu des überkritischen Fluids zusätzliche Schritte umfasst, wobei die zusätzlichen Schritte die Schritte umfassen:
(a) Entfernen der Ester aus dem Produktgemisch der Schritte (i) und (ii),
(b) Verseifen der Fraktion des Produktgemischs der Schritte (i) und (ii), wobei die Fraktion die nicht veresterten Bestandteile enthält;
(c) Entfernen der verseiften Produkte, wobei eine Fraktion erhalten wird, welche nicht verseifbare Produkte enthält;
worin die Substanzen, welche die Quelle der Vitamin E-Isomere bilden, welche auf die selektiven Adsorbtionsmittel aufgebracht werden, die Fraktion umfassen, welche die nicht verseifbaren Produkte aus dem obigen Schritt (c) enthält.

14. Verfahren zur chromatographischen Isolierung von Vitamin E-Isomeren nach Anspruch 1, wobei das Verfahren den zusätzlichen Schritt (die zusätzlichen Schritte) des Konzentrierens der Verbindungen, welche Vitamin E umfassen, durch ein beliebiges oder verschiedene Verfahren einbezieht, einschließlich einer Chromatogaphie, welche mit der Verwendung des Milieus eines überkritischen Fluids verbunden ist.

15. Verfahren zur chromatographischen Isolierung von Vitamin E-Isomeren nach Anspruch 1, wobei das Verfahren den zusätzlichen Schritt (die zusätzlichen Schritte) des Extrahierens eines Vitamin E-Konzentrats aus den Verbindungen, welche Vitamin E umfassen, durch ein beliebiges oder verschiedene Verfahren einbezieht, einschließlich einer chromatographischen Isolierung, welche mit der Verwendung des Milieus eines überkritischen Fluids verbunden ist.

16. Verfahren zur chromatographischen Isolierung von Vitamin E-Isomeren nach Anspruch 1, welches verwendet wird, um Vitamin E-Isomere aus Sojabohnenöl, Maisöl, Canolaöl, Olivenöl, Baumwollsamenöl und/oder Sonnenblumenkernöl abzutrennen.

17. Verfahren zur chromatographischen Isolierung von Vitamin E-Isomeren nach Anspruch 16, worin die Vitamin E-Isomere in α-T-, β-T-, γ-T- und δ-T-Isomere aufgetrennt werden.

18. Verfahren zur chromatographischen Isolierung von Vitamin E-Isomeren nach Anspruch 1, welches verwendet wird, um Vitamin E-Isomere aus Erdnussöl abzutrennen.

19. Verfahren zur chromatographischen Isolierung von Vitamin E-Isomeren nach Anspruch 18, worin die Vitamin E-Isomere in α-T-, β-T-, γ-T- und δ-T-Isomere aufgetrennt werden.

20. Verfahren zur chromatographischen Isolierung von Vitamin E-Isomeren nach Anspruch 1, welches verwendet wird, um Vitamin E-Isomere aus Reiskleieöl abzutrennen.

21. Verfahren zur chromatographischen Isolierung von Vitamin E-Isomeren nach Anspruch 20, worin die Vitamin E-Isomere in α-T-, γ-T-, α-T₃- und γ-T₃-Isomere aufgetrennt werden.

22. Verfahren zur chromatographischen Isolierung von Vitamin E-Isomeren nach Anspruch 1, welches verwendet wird, um Vitamin E-Isomere aus rohem Palmöl, Palmfettsäuredestillat und/oder gepresstem Palmfaseröl abzutrennen.

23. Verfahren zur chromatographischen Isolierung von Vitamin E-Isomeren nach Anspruch 22, worin die Vitamin E-Isomere in α-T-, γ-T₃-, δ-T₃- und α-T₃-Isomere aufgetrennt werden.

24. Verfahren zur chromatographischen Isolierung von Vitamin E-Isomeren nach Anspruch 5, worin der Alkohol Ethanol ist.

25. Verfahren zur chromatographischen Isolierung von Vitamin E-Isomeren nach Anspruch 1, worin das verwendete überkritische Lösungsmittel Propan, Propen oder Ethylen ist.

26. Verfahren zur chromatographischen Isolierung von Vitamin E-Isomeren nach Anspruch 1, worin die veresterten und umgeesterten Produkte aus den Schritten (i) und (ii) vor den Schritten (iii) und (iv) durch Vakuumdestillation entfernt werden.

## Patentansprüche (Patentansprüche für folgende(n) Vertragsstaat(en): DE, CH, GB)

1. Verfahren zur chromatographischen Isolierung von Vitamin E-Isomeren aus Verbindungen, welche Vitamin E-Isomere umfassen, wobei das Verfahren die Schritte umfasst:
(i) Verestern von freien Fettsäurebestandteilen der Verbindungen mit einem oder mehreren einwertigen Alkoholen zu Estern;
(ii) Umestern von Glyceridbestandteilen der Verbindungen mit einem oder mehreren einwertigen Alkoholen zu Estern;
(iii) Einbringen des Produktgemischs aus den Schritten (i) und (ii) auf selektive Adsorbtionsmittel, um die Adsorption der Vitamin E-Isomere zu bewirken; und anschließend
(iv) Ausführen einer individuellen Desorption der adsorbierten Vitamin E-Isomere, worin die Adsorption und/oder Desorption der Vitamin E-Isomere im Milieu eines überkritischen Fluids durchgeführt werden, worin die Verbindungen, welche Vitamin E-Isomere umfassen, keine rohen Palmöle und/oder Palmölprodukte sind.

2. Verfahren zur chromatographischen Isolierung nach Anspruch 1, worin die selektiven Adsorptionsmittel Silicagel und/oder Umkehrphasen-C18-Silicagel umfassen.

3. Verfahren zur chromatographischen Isolierung nach Anspruch 1, worin die Adsorption/Desorption der Vitamin E-Isomere im Milieu eines überkritischen Fluids durchgeführt werden, welches eine wirksame Menge CO₂ als ein Lösungsmittel umfasst.

4. Verfahren zur chromatographischen Isolierung nach Anspruch 1, worin die Adsorption/Desorption der Vitamin E-Isomere im Milieu eines überkritischen Fluids durchgeführt werden, welches eine wirksame Menge einer Kombination von CO₂ und einem Schleppmittel umfasst.

5. Verfahren zur chromatographischen Isolierung nach Anspruch 4, worin das Schleppmittel einen Alkohol umfasst.

6. Verfahren zur chromatographischen Isolierung nach einem der Ansprüche 3-5, worin die Bedingungen des überkritischen Fluids Betriebstemperaturen von T=30-100°C und Drücke von P=50-600 kg/cm² umfassen.

7. Verfahren zur chromatographischen Isolierung von Vitamin E-Isomeren nach Anspruch 1, worin die in den Schritten (i) und (ii) gebildeten Ester entfernt werden, um ein Vitamin E-Konzentrat zu bilden,
worin die Substanzen, welche die Quelle der Vitamin E-Isomere bilden, welche in die selektiven Adsorptionsmittel eingebracht werden, das Vitamin E-Konzentrat umfassen.

8. Verfahren zur chromatographischen Isolierung nach Anspruch 1, worin:
Schritt (i) mittels Verwendung eines festen Alkalimetallbisulfats, einer Sulfatsäure und/oder eines stark sauren Ionenaustauschharzes als ein Katalysator durchgeführt wird;
Schritt (ii) mittels Verwendung eines basischen Katalysators durchgeführt wird;
oder worin die Veresterung und/oder Umesterung mittels Verwendung eines Enzyms durchgeführt werden.

9. Verfahren zur chromatographischen Isolierung nach Anspruch 1, worin die Veresterung und/oder Umesterung die Verwendung von einem oder mehreren C₁-C₈ Alkoholen umfasst.

10. Verfahren zur chromatographischen Isolierung nach Anspruch 9, worin die Veresterung und/oder Umesterung die Verwendung von Methanol umfasst.

11. Verfahren zur chromatographischen Isolierung nach Anspruch 5 oder 6,
worin das Schleppmittel einen Alkylalkohol umfasst.

12. Verfahren zur chromatographischen Isolierung von Vitamin E-Isomeren nach Anspruch 1, worin die Verbindungen, welche Vitamin E umfassen, rohes Pflanzenöl, Pflanzenölprodukte und/oder Pflanzenölnebenprodukte einschließen.

13. Verfahren zur chromatographischen Isolierung von Vitamin E-Isomeren nach Anspruch 1, wobei das Verfahren vor den Adsorptions- und Desorptionsschritten im Milieu des überkritischen Fluids zusätzliche Schritte umfasst, wobei die zusätzlichen Schritte die Schritte umfassen:
(a) Entfernen der Ester aus dem Produktgemisch der Schritte (i) und (ii),
(b) Verseifen der Fraktion des Produktgemischs der Schritte (i) und (ii), wobei die Fraktion die nicht veresterten Bestandteile enthält;
(c) Entfernen der verseiften Produkte, wobei eine Fraktion erhalten wird, welche nicht verseifbare Produkte enthält;
worin die Substanzen, welche die Quelle der Vitamin E-Isomere bilden, welche auf die selektiven Adsorbtionsmittel aufgebracht werden, die Fraktion umfassen, welche die nicht verseifbaren Produkte aus dem obigen Schritt (c) enthält.

14. Verfahren zur chromatographischen Isolierung von Vitamin E-Isomeren nach Anspruch 1, wobei das Verfahren den zusätzlichen -Schritt (die zusätzlichen Schritte) des Konzentrierens der Verbindungen, welche Vitamin E umfassen, durch ein beliebiges oder verschiedene Verfahren einbezieht, einschließlich einer Chromatogaphie, welche mit der Verwendung des Milieus eines überkritischen Fluids verbunden ist.

15. Verfahren zur chromatographischen Isolierung von Vitamin E-Isomeren nach Anspruch 1, wobei das Verfahren den zusätzlichen Schritt (die zusätzlichen Schritte) des Extrahierens eines Vitamin E-Konzentrats aus den Verbindungen, welche Vitamin E umfassen, durch ein beliebiges oder verschiedene Verfahren einbezieht, einschließlich einer chromatographischen Isolierung, welche mit der Verwendung des Milieus eines überkritischen Fluids verbunden ist.

16. Verfahren zur chromatographischen Isolierung von Vitamin E-isomeren nach Anspruch 1, welches verwendet wird, um Vitamin E-Isomere aus Sojabohnenöl, Maisöl, Canoiaöl, Olivenöl, Baumwollsamenöl und/oder Sonnenblumenkernöl abzutrennen.

17. Verfahren zur chromatographischen Isolierung von Vitamin E-Isomeren nach Anspruch 16, worin die Vitamin E-Isomere in α-T-, β-T-, γ-T- und δ-T-Isomere aufgetrennt werden.

18. Verfahren zur chromatographischen Isolierung von Vitamin E-Isomeren nach Anspruch 1, welches verwendet wird, um Vitamin E-Isomere aus Erdnussöl abzutrennen.

19. Verfahren zur chromatographischen Isolierung von Vitamin E-Isomeren nach Anspruch 18, worin die Vitamin E-Isomere in α-T-, β-T-, γ-T- und δ-T-Isomere aufgetrennt werden.

20. Verfahren zur chromatographischen Isolierung von Vitamin E-Isomeren nach Anspruch 1, welches verwendet wird, um Vitamin E-isomere aus Reiskleieöl abzutrennen.

21. Verfahren zur chromatographischen Isolierung von Vitamin E-Isomeren nach Anspruch 20, worin die Vitamin E-Isomere in γ-T-, α-T-, α-T₃- und γ-T₃-Isomere aufgetrennt werden.

22. Verfahren zur chromatographischen Isolierung von Vitamin E-Isomeren nach Anspruch 1, worin das verwendete überkritische Lösungsmittel Propan, Propen oder Ethylen ist.

23. Verfahren zur chromatographischen Isolierung von Vitamin E-Isomeren nach Anspruch 5, worin der Alkohol Ethanol ist.

24. Verfahren zur chromatographischen Isolierung von Vitamin E-Isomeren nach Anspruch 1, worin die veresterten und umgeesterten Produkte aus den Schritten (i) und (ii) vor den Schritten (iii) und (iv) durch Vakuumdestillation entfernt werden.

## Revendications (Revendications pour l'(les) Etat(s) contractant(s) suivant(s): FR)

1. Procédé d'isolement chromatographique d'isomères de vitamine E à partir de matières comprenant des isomères de vitamine E, ledit procédé comprenant les étapes suivantes :
(i) estérifier les composants acides gras libres desdites matières pour former des esters avec un ou plusieurs monoalcools ;
(ii) trans-estérifier les composants glycéridiques desdites matières pour former des esters avec un ou plusieurs monoalcools ;
(iii) introduire le mélange de produits des étapes (i) et (ii) sur des adsorbants sélectifs pour effectuer l'adsorption d'isomères de vitamine E ; et ensuite
(iv) effectuer une désorption individuelle des isomères de vitamine E adsorbés, l'adsorption et/ou la désorption des isomères de vitamine E étant effectuées dans un environnement de fluide supercritique.

2. Procédé d'isolement chromatographique selon la revendication 1, dans lequel les adsorbants sélectifs comprennent du gel de silice et/ou un gel de silice à phase inversée en C18.

3. Procédé d'isolement chromatographique selon la revendication 1, dans lequel l'adsorption/la désorption des isomères de vitamine E sont effectuées dans un environnement de fluide supercritique comprenant une quantité efficace de CO₂ comme solvant.

4. Procédé d'isolement chromatographique selon la revendication 1, dans lequel l'adsorption/la désorption des isomères de vitamine E sont effectuées dans un environnement de fluide supercritique comprenant une quantité efficace d'une association de CO₂ et d'un entraîneur.

5. Procédé d'isolement chromatographique selon la revendication 4, dans lequel l'entraîneur comprend un alcool.

6. Procédé d'isolement chromatographique selon l'une quelconque des revendications 3 à 5, dans lequel les conditions de fluide supercritique comprennent des températures opératoires de T = 30-100°C et des pressions de P = 50-600 kg/cm².

7. Procédé d'isolement chromatographique d'isomères de vitamine E selon la revendication 1, dans lequel les esters formés dans les étapes (i) et (ii) sont éliminés pour former un concentré de vitamine E,
dans lequel les substances qui constituent la source des isomères de vitamine E, introduites dans les adsorbants sélectifs, comprennent ledit concentré de vitamine E.

8. Procédé d'isolement chromatographique selon la revendication 1, dans lequel :
l'étape (i) est effectuée en utilisant un bisulfate de métal alcalin solide, un sulfate acide et/ou une résine échangeuse d'ions fortement acide comme catalyseur ;
l'étape (ii) est effectuée en utilisant un catalyseur basique ;
ou dans lequel
l'estérification et/ou la trans-estérification sont effectuées en utilisant une enzyme.

9. Procédé d'isolement chromatographique selon la revendication 1, dans lequel l'estérification et/ou la trans-estérification comprennent l'utilisation d'un ou de plusieurs alcools en C₁-C₈.

10. Procédé d'isolement chromatographique selon la revendication 9, dans lequel l'estérification et/ou la trans-estérification comprennent l'utilisation de méthanol.

11. Procédé d'isolement chromatographique selon la revendication 5 ou 6, dans lequel l'entraîneur comprend un alcool alkylique.

12. Procédé d'isolement chromatographique d'isomères de vitamine E selon la revendication 1, dans lequel les matières comprenant la vitamine E comprennent une huile végétale brute, des produits d'huile végétale et/ou des sous-produits d'huile végétale.

13. Procédé d'isolement chromatographique d'isomères de vitamine E selon la revendication 1, ledit procédé comportant des étapes supplémentaires avant les stades d'adsorption et de désorption dans l'environnement de fluide supercritique, lesdites étapes supplémentaires comprenant les étapes suivantes :
(a) éliminer les esters du mélange de produits des étapes (i) et (ii),
(b) saponifier la fraction du mélange de produits des étapes (i) et (ii), laquelle fraction contient les composants non estérifiés ;
(c) éliminer les produits saponifiés, pour obtenir ainsi une fraction contenant les produits insaponifiables ;
dans lequel les substances qui constituent la source des isomères de vitamine E, introduites sur les adsorbants sélectifs, comprennent la fraction contenant les produits insaponifiables provenant de l'étape (c) ci-dessus.

14. Procédé d'isolement chromatographique d'isomères de vitamine E selon la revendication 1, ledit procédé comprenant la ou les étapes supplémentaires consistant à concentrer les matières comprenant de la vitamine E par n'importe quelle ou diverses techniques incluant une chromatographie comportant l'utilisation de l'environnement de fluide supercritique.

15. Procédé d'isolement chromatographique d'isomères de vitamine E selon la revendication 1, ledit procédé comprenant la ou les étapes supplémentaires consistant à extraire un concentré de vitamine E des matières comprenant de la vitamine E par n'importe quelle ou diverses techniques incluant un isolement chromatographique comportant l'utilisation d'un environnement de fluide supercritique.

16. Procédé d'isolement chromatographique d'isomères de vitamine E selon la revendication 1, qui est utilisé pour séparer les isomères de vitamine E à partir d'huile de soja, d'huile de maïs, d'huile de canola, d'huile d'olive, d'huile de coton et/ou d'huile de tournesol.

17. Procédé d'isolement chromatographique d'isomères de vitamine E selon la revendication 16, dans lequel lesdits isomères de vitamine E sont séparés en isomères α-T, β-T, γ-T et δ-T.

18. Procédé d'isolement chromatographique d'isomères de vitamine E selon la revendication 1, qui est utilisé pour séparer les isomères de vitamine E à partir d'huile d'arachide.

19. Procédé d'isolement chromatographique d'isomères de vitamine E selon la revendication 18, dans lequel les isomères de vitamine E sont séparés en isomères α-T, β-T, γ-T et δ-T.

20. Procédé d'isolement chromatographique d'isomères de vitamine E selon la revendication 1, qui est utilisé pour séparer les isomères de vitamine E à partir d'huile de son de riz.

21. Procédé d'isolement chromatographique d'isomères de vitamine E selon la revendication 20, dans lequel les isomères de vitamine E sont séparés en α-T, γ-T, α-T₃ et γ-T₃.

22. Procédé d'isolement chromatographique d'isomères de vitamine E selon la revendication 1, qui est utilisé pour séparer les isomères de vitamine E à partir d'huile de palme brute, de distillat d'acide gras de palme et/ou d'huile de fibre pressée de palme.

23. Procédé d'isolement chromatographique d'isomères de vitamine E selon la revendication 22, dans lequel les isomères de vitamine E sont séparés en isomères α-T, γ-T₃, δ-T₃ et α-T₃.

24. Procédé d'isolement chromatographique d'isomères de vitamine E selon la revendication 5, dans lequel l'alcool est l'éthanol.

25. Procédé d'isolement chromatographique d'isomères de vitamine E selon la revendication 1, dans lequel le solvant supercritique utilisé est le propane, le propène ou l'éthylène.

26. Procédé d'isolement chromatographique selon la revendication 1, dans lequel les produits estérifiés et trans-estérifiés des étapes (i) et (ii) sont éliminés par distillation sous vide, avant les étapes (iii) et (iv).

## Revendications (Revendications pour l'(les) Etat(s) contractant(s) suivant(s): DE, CH, GB)

1. Procédé d'isolement chromatographique d'isomères de vitamine E à partir de matières comprenant des isomères de vitamine E, ledit procédé comprenant les étapes suivantes :
(i) estérifier les composants acides gras libres desdites matières pour former des esters avec un ou plusieurs monoalcools ;
(ii) trans-estérifier les composants glycéridiques desdites matières pour former des esters avec un ou plusieurs monoalcools ;
(iii) introduire le mélange de produits des étapes (i) et (ii) sur des adsorbants sélectifs pour effectuer l'adsorption d'isomères de vitamine E ; et ensuite
(iv) effectuer une désorption individuelle des isomères de vitamine E adsorbés, l'adsorption et/ou la désorption des isomères de vitamine E étant effectuées dans un environnement de fluide supercritique,
les matières comprenant des isomères de vitamine E n'étant pas des huiles de palme brutes et/ou des produits d'huile de palme.

2. Procédé d'isolement chromatographique selon la revendication 1, dans lequel les adsorbants sélectifs comprennent du gel de silice et/ou un gel de silice à phase inversée en C18.

3. Procédé d'isolement chromatographique selon la revendication 1, dans lequel l'adsorption/la désorption des isomères de vitamine E sont effectuées dans un environnement de fluide supercritique comprenant une quantité efficace de CO₂ comme solvant.

4. Procédé d'isolement chromatographique selon la revendication 1, dans lequel l'adsorption/la désorption des isomères de vitamine E sont effectuées dans un environnement de fluide supercritique comprenant une quantité efficace d'une association de CO₂ et d'un entraîneur.

5. Procédé d'isolement chromatographique selon la revendication 4, dans lequel l'entraîneur comprend un alcool.

6. Procédé d'isolement chromatographique selon l'une quelconque des revendications 3 à 5, dans lequel les conditions de fluide supercritique comprennent des températures opératoires de T = 30-100°C et des pressions de P = 50-600 kg/cm².

7. Procédé d'isolement chromatographique d'isomères de vitamine E selon la revendication 1, dans lequel les esters formés dans les étapes (i) et (ii) sont éliminés pour former un concentré de vitamine E,
dans lequel les substances qui constituent la source des isomères de vitamine E, introduites dans les adsorbants sélectifs, comprennent ledit concentré de vitamine E.

8. Procédé d'isolement chromatographique selon la revendication 1, dans lequel :
l'étape (i) est effectuée en utilisant un bisulfate de métal alcalin solide, un sulfate acide et/ou une résine échangeuse d'ions fortement acide comme catalyseur ;
l'étape (ii) est effectuée en utilisant un catalyseur basique ;
ou dans lequel
l'estérification et/ou la trans-estérification sont effectuées en utilisant une enzyme.

9. Procédé d'isolement chromatographique selon la revendication 1, dans lequel l'estérification et/ou la trans-estérification comprennent l'utilisation d'un ou de plusieurs alcools en C₁-C₈.

10. Procédé d'isolement chromatographique selon la revendication 9, dans lequel l'estérification et/ou la trans-estérification comprennent l'utilisation de méthanol.

11. Procédé d'isolement chromatographique selon la revendication 5 ou 6, dans lequel l'entraîneur comprend un alcool alkylique.

12. Procédé d'isolement chromatographique d'isomères de vitamine E selon la revendication 1, dans lequel les matières comprenant la vitamine E comprennent une huile végétale brute, des produits d'huile végétale et/ou des sous-produits d'huile végétale.

13. Procédé d'isolement chromatographique d'isomères de vitamine E selon la revendication 1, ledit procédé comportant des étapes supplémentaires avant les stades d'adsorption et de désorption dans l'environnement de fluide supercritique, lesdites étapes supplémentaires comprenant les étapes suivantes :
(a) éliminer les esters du mélange de produits des étapes (i) et (ii),
(b) saponifier la fraction du mélange de produits des étapes (i) et (ii), laquelle fraction contient les composants non estérifiés ;
(c) éliminer les produits saponifiés, pour obtenir ainsi une fraction contenant les produits insaponifiables ;
dans lequel les substances qui constituent la source des isomères de vitamine E, introduites sur les adsorbants sélectifs, comprennent la fraction contenant les produits insaponifiables provenant de l'étape (c) ci-dessus.

14. Procédé d'isolement chromatographique d'isomères de vitamine E selon la revendication 1, ledit procédé comprenant la ou les étapes supplémentaires consistant à concentrer les matières comprenant de la vitamine E par n'importe quelle ou diverses techniques incluant une chromatographie comportant l'utilisation de l'environnement de fluide supercritique.

15. Procédé d'isolement chromatographique d'isomères de vitamine E selon la revendication 1, ledit procédé comprenant la ou les étapes supplémentaires consistant à extraire un concentré de vitamine E des matières comprenant de la vitamine E par n'importe quelle ou diverses techniques incluant un isolement chromatographique comportant l'utilisation d'un environnement de fluide supercritique.

16. Procédé d'isolement chromatographique d'isomères de vitamine E selon la revendication 1, qui est utilisé pour séparer les isomères de vitamine E à partir d'huile de soja, d'huile de maïs, d'huile de canola, d'huile d'olive, d'huile de coton et/ou d'huile de tournesol.

17. Procédé d'isolement chromatographique d'isomères de vitamine E selon la revendication 16, dans lequel lesdits isomères de vitamine E sont séparés en isomères α-T, β-T, γ-T et δ-T.

18. Procédé d'isolement chromatographique d'isomères de vitamine E selon la revendication 1, qui est utilisé pour séparer les isomères de vitamine E à partir d'huile d'arachide.

19. Procédé d'isolement chromatographique d'isomères de vitamine E selon la revendication 18, dans lequel les isomères de vitamine E sont séparés en isomères α-T, β-T, γ-T et δ-T.

20. Procédé d'isolement chromatographique d'isomères de vitamine E selon la revendication 1, qui est utilisé pour séparer les isomères de vitamine E à partir d'huile de son de riz.

21. Procédé d'isolement chromatographique d'isomères de vitamine E selon la revendication 20, dans lequel les isomères de vitamine E sont séparés en γ-T, α-T, α-T₃ et γ-T₃.

22. Procédé d'isolement chromatographique d'isomères de vitamine E selon la revendication 1, dans lequel le solvant supercritique utilisé est le propane, le propène ou l'éthylène.

23. Procédé d'isolement chromatographique d'isomères de vitamine E selon la revendication 5, dans lequel l'alcool est l'éthanol.

24. Procédé d'isolement chromatographique selon la revendication 1, dans lequel les produits estérifiés et trans-estérifiés des étapes (i) et (ii) sont éliminés par distillation sous vide, avant les étapes (iii) et (iv).
